# EUROPEAN PATENT APPLICATION

(11) **EP 1 905 432 A1**
(43) Date of publication of application: **02.04.2008**
(21) Application number: 07252670.0
(22) Date of filing: 02.07.2007
(51) Int. Cl.: A61K 9/51, A61K 9/20

(54) **Nanoparticulate and controlled release compositions comprising a cephalosporin**

(30) Priority: 30.06.2006 US 478891
(71) Applicant: ELAN CORPORATION, Plc, Dublin 2 (IE)
(72) Inventor: Jenkins, Scott, Downingtown, PA 19335 (US); Liversidge, Gary, West Chester, PA 19380 (US); Devane, John G., Athlone, County Roscommon (IE); Rekhi, Gurvinder Singh, Suwanee, GA 30024 (US); Stark, Paul, Glasson, County Westmeath (IE); Fanning, Niall M.M., Swords, County Dublin (IE)
(74) Representative: Stoner, Gerard Patrick

(57) **Abstract**

The present invention provides a composition comprising cephalosporin useful in the treatment and prevention of a bacterial infection. In one embodiment, the composition comprises nanoparticulate particles comprising cephalosporin and at least one surface stabilizer. The nanoparticulate particles have an effective average particle size of less than about 2000 nm. In another embodiment, the composition comprises a modified release composition that, upon admiriistration to a patient, delivers cephalosporin in a bimodal, multimodal or continuous manner. The invention also relates to dosage forms containing such compositions, and to methods for the treatment and prevention of a bacterial infection.

## Description

The present invention relates to compositions that are useful for the prevention and treatment of bacterial infections. In particular, the present invention relates to compositions comprising a cephalosporin, for example, cefdinir, or a salt, derivative, prodrug, or polymorph thereof. In one aspect of the invention, the composition is in nanoparticulate form and comprises also at least one surface stabilizer. In another aspect, the present invention relates to novel dosage forms for the controlled delivery of a cephalosporin, for example, cefdinir or a salt, derivative, prodrug, or polymorph thereof, and to methods for the prevention and treatment of bacterial infections.

### BACKGROUND

Antibiotics are powerful bacteria-killing drugs used to treat bacterial infections in humans and other mammals. There are hundreds of antibiotics currently in use, most tailored to treat a specific kind of bacterial infection. Beta-lactam antibiotics, which are named for the beta-lactam ring in their chemical structure, include the penicillins, cephalosporins and related compounds. These agents are active against many gram-positive, gram-negative and anaerobic organisms. The beta-lactam antibiotics exert their effect by interfering with the structural crosslinking of peptidoglycans in bacterial cell walls. Because many of these drugs are well absorbed after oral administration, they are clinically useful in the outpatient setting.

The cephalosporin beta-lactam antibiotics are a group of semi-synthetic derivatives of cephalosporin C, an antimicrobial agent of fungal origin. They are structurally and pharmacologically related to the penicillins. The cephalosporin ring structure is derived from 7-amino-cephalosporanic acid (7-ACA) while the penicillins are derived from 6-aminopenicillanic acid (6-APA). Both structures contain the basic beta-lactam ring but the cephalosporin structure allows for more gram negative activity than the penicillins and aminocillins. Substitution of different side chains on the cephalosporin ring allows for variation in the spectrum of activity and duration of action.

Cephalosporins are grouped into "generations" by their antimicrobial properties. The first cephalosporins were designated first generation while later, more extended spectrum cephalosporins were classified as second generation cephalosporins. Currently, three generations of cephalosporins are recognized and a fourth has been proposed. Significantly, each newer generation of cephalosporins has greater gram negative antimicrobial properties than the preceding generation. Conversely, the "older" generations of cephalosporins have greater gram positive coverage than the "newer" generations.

Cephalosporins are used to treat infections in many different parts of the body. They are sometimes given with other antibiotics. Some cephalosporins given by injection are also used to prevent infections before, during, and after surgery.

Cefdinir is a third generation cephalosporin with activity against Gram-positive and Gram-negative bacteria. In particular, cefdinir has increased activity against Gram-positive bacteria, as compared to other third generation cephalosporins.

Cefdinir has the chemical name [6R-[6, 7 (Z)]]-7-[[(2-amino-4-thiazolyl) (hydroxylimino)acetyl]amino]-3-ethenyl-8-oxo-5-thia-1-azabicyclo[4.2.0]oct-2-ene-2-carboxylic acid. Its empirical formula is C₁₄H₁₃N₅O₅S₂ and its molecular weight is 395.42. The structural formula of cefdinir is shown below: 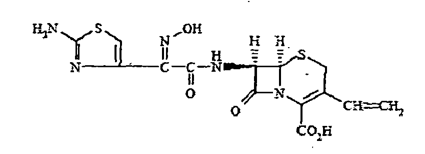 Cefdinir is a white to slightly brownish-yellow solid. Cefdinir is practically insoluble in water.

Cefdinir is offered under the registered trademark OMNICEF® by Abbott Laboratories of North Chicago, IL as well as CEFZON® by Fujisawa Pharmaceutical Co., Ltd. of Japan. Both OMNICEF® and CEFZON® are indicated for the treatment of various bacterial infections. Cefdinir is administered orally as a capsule or oral suspension and is given two to three times daily.

Cefdinir is described, for example, in U.S. Pat. Nos. 6,093,814; 6,350,869; 6,369,050; and 6,423,341. These patents are hereby incorporated by reference herein.

Cephalosporins have high therapeutic value for the treatment of patients suffering from bacterial infection. However, given the need to take cephalosporins such as cefdinir two or three times a day, strict patient compliance is a critical factor in the efficacy of cephalosporins in the treatment of bacterial infection. Moreover, such frequent administration often requires the attention of health care workers and contributes to the high cost associated with treatments involving cephalosporin. Thus, there is a need in the art for cephalosporin compositions which address these and other problems associated with their use in the treatment of bacterial infection.

The present invention fulfills such a need by providing nanoparticulate cephalosporin compositions, preferably compositions comprising cefdinir, or a salt, derivative, prodrug, or polymorph thereof, in which the poor bioavailability of cephalosporin is improved. The invention also provides controlled release compositions of nanoparticulate cephalosporin, preferably cefdinir or a salt, derivative, prodrug, or polymorph thereof, which can reduce or eliminate the need to take cephalosporins such as cefdinir two or three times a day.

Some aspects of the invention are set out in the claims.

One aspect of the invention encompasses a nanoparticulate composition comprising: (A) a cephalosporin; and (B) at least one surface stabilizer. The surface stabilizer can be adsorbed on or associated with the surface of the nanoparticulate particles. The nanoparticulate particles have an effective average particle size of less than about 2,000 nm. The cephalosporin may, for example, be cefdinir or a salt, derivative, prodrug, or polymorph thereof. The nanoparticulate composition may optionally comprise one or more additional active ingredients useful in the prevention and treatment of a bacterial infection and/or one or more pharmaceutically acceptable excipients. The administration of the nanoparticulate composition to a subject in a fed or fasted state may be bioequivalent and may exhibit similar pharmacokinetics.

Another aspect herein, compatible with that above, relates to modified release compositions having a first component comprising a first population of active ingredient-containing particles and at least one further component comprising a further population of active ingredient-containing particles, wherein each component has a different rate and/or duration of release and wherein at least one of said components comprises a cephalosporin, for example cefdinir or a salt, derivative, prodrug, or polymorph thereof. The particles of at least one further component are provided in a modified release (MR) form such as, for example, particles coated with a modified release coating or comprising or incorporated in a modified release matrix material. Upon oral administration to a patient, the composition releases the active ingredient(s) in a bimodal or multimodal manner. The components may optionally comprise one or more additional active ingredients useful in the treatment of bacterial infections and/or one or more pharmaceutically acceptable excipients. In a preferred embodiment, at least some of the particles comprise nanoparticles which comprise a cephalosporin, for example cefdinir or a salt, derivative, prodrug, or polymorph thereof. In another preferred embodiment, at least some of the particles are themselves nanoparticles which comprise a cephalosporin, for example cefdinir or a salt, derivative, prodrug, or polymorph thereof.

The first component of the modified release composition may exhibit a variety of release profiles including profiles in which substantially all of the active ingredient contained in the first component is released rapidly upon administration of the dosage form, released rapidly but after a time delay (delayed release), or released slowly over time. In one embodiment, the active ingredient contained in the first component of the dosage form is released rapidly upon administration to a patient. As used herein, "released rapidly" includes release profiles in which at least about 80% of the active ingredient of a component of the dosage form is released within about an hour after administration, the term "delayed release" includes release profiles in which the active ingredient of a component of the dosage form is released (rapidly or slowly) after a time delay, and the terms "controlled release" and "extended release" include release profiles in which at least about 80% of the active ingredient contained in a component of the dosage form is released slowly.

A second component of the modified release composition may also exhibit a variety of release profiles including an immediate release profile, a delayed release profile or a controlled release profile. In one embodiment, the second component exhibits a delayed release profile in which the active ingredient of the component is released after a time delay. In another embodiment, the second component exhibits a controlled release profile in which the active ingredient of the component is released over a period of about 12 to about 24 hours after administration.

In two-component embodiments in which the components exhibit different release profiles, the release profile of the active ingredients from the composition is bimodal. In embodiments in which the first component exhibits an immediate release profile and the second component exhibits a delayed release profile, there is a lag time between the release of active ingredient from the first component and the release of the active ingredient from the second component. The duration of the lag time may be varied by altering the amount and/or composition of the modified release coating or by altering the amount and/or composition of the modified release matrix material utilized to achieve the desired release profile. Thus, the duration of the lag time can be designed to promote or mimic a desired plasma profile.

In embodiments in which the first component exhibits an immediate release profile and the second component exhibits a controlled release profile, the active ingredients in the first and second components are released over different time periods. In such embodiments, the immediate release component serves to hasten the onset of action by minimizing the time from administration to a therapeutically effective plasma concentration level, and the one or more subsequent components serve to minimize the variation in plasma concentration levels and/or maintain a therapeutically effective plasma concentration throughout the dosing interval. In one such embodiment, the active ingredient in the first component is released rapidly and the active ingredient in the second component is released within a period of about 12 hours after administration. In another such embodiment, the active ingredient in the first component is released rapidly and the active ingredient in the second component is released within a period of about 24 hours after administration. In yet another such embodiment, the active ingredient in the first component is released rapidly and the active ingredient in the second component is released over a period of about 12 hours after administration. In still another such embodiment, the active ingredient in the first component is released rapidly and the active ingredient in the second component is released over a period of about 24 hours after administration. In yet another such embodiment, the active ingredient in the first component is released rapidly and the active ingredient in the second component is released over a period of at least about 12 hours after administration. In still another such embodiment, the active ingredient in the first component is released rapidly and the active ingredient in the second component is released over a period of at least about 24 hours after administration.

The plasma profile produced by the administration of dosage forms of the present invention which comprise an immediate release component and at least one modified release component can be substantially similar to the plasma profile produced by the administration of two or more IR dosage forms given sequentially, or to the plasma profile produced by the administration of separate IR and MR dosage forms. The modified release compositions as proposed herein are particularly useful for administering a cephalosporin, for example, cefdinir, or a salt, derivative, prodrug, or polymorph thereof, which is normally administered two times daily. In one embodiment of the present invention, the composition delivers the cephalosporin in a bimodal manner. Upon administration, such a composition produces a plasma profile which substantially mimics that obtained by the sequential administration of two IR doses of cephalosporin in accordance with a typical treatment regimen.

According to another aspect of the present invention, the composition can be designed to produce a plasma profile that minimizes or eliminates the variations in plasma concentration levels associated with the administration of two or more IR dosage forms given sequentially. In such embodiments, the composition may be provided with an immediate release component to hasten the onset of action by minimizing the time from administration to a therapeutically effective plasma concentration level, and at least one modified release component to maintain a therapeutically effective plasma concentration level throughout the dosing interval. The cephalosporin, for example cefdinir or a salt, derivative, prodrug, or polymorph thereof, may be contained in nanoparticulate particles which comprise also at least one surface stabilizer.

Modified release compositions similar to those disclosed herein are disclosed and claimed in the United States Patent Nos. 6,228,398 and 6,730,325 to Devane et al.

A further aspect herein relates to dosage forms made from the compositions disclosed herein. In one embodiment, the dosage form is a solid oral dosage form comprising the modified release composition of the present invention. The oral dosage form may utilize, for example, erodable formulations, diffusion controlled formulations and osmotic controlled formulations. In such embodiments, the total dose contained in the dosage form may be released in a pulsatile or continuous manner. In one such embodiment, a portion of the total dose is released immediately to allow for rapid onset of effect, and the remainder of the total dose is release after a lag time or over a period of time up to about 24 hours.

A further aspect herein is a method of making a nanoparticulate composition comprising a cephalosporin, for example cefdinir or a salt, derivative, prodrug, or polymorph thereof. Such method comprises the step of contacting nanoparticulate particles comprising the cephalosporin with at least one surface stabilizer for a period of time and under conditions sufficient to provide a stabilized nanoparticulate composition comprising cephalosporin.

A further aspect is that the present compositions or dosage forms are for methods of medical treatment, including but not limited to the prevention and treatment of a bacterial infection. Such methods comprise the step of administering to a subject a therapeutically effective amount of a composition, for example, a nanoparticulate composition comprising cephalosporin, for example cefdinir or a salt, derivative, prodrug, or polymorph thereof.

We find that the present compositions and dosage forms are useful in reducing the required dosing frequency thereby increasing patient convenience and improving patient compliance and, therefore, the therapeutic outcome for all treatments requiring cephalosporin, for example cefdinir or a salt, derivative, prodrug, or polymorph thereof, including but not limited to, the prevention and treatment of a bacterial infection. This approach can replace conventional cephalosporin dosage forms, which are administered multiple times daily.

Both the foregoing general description and the following detailed description are exemplary and explanatory and are intended to provide further explanation of the invention as claimed. Other objects, advantages, and novel features will be readily apparent to those skilled in the art from the following detailed description.

### FURTHER EXPLANATIONS; OPTIONS AND PREFERENCES

The present application uses several definitions as set forth below and throughout the application.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term (and the particular term is itself disclosed).

As used herein, "therapeutically effective amount" means the dosage that provides the specific pharmacological response for which the active agent is administered in a significant number of subjects in need of the relevant treatment. It is emphasized that a therapeutically effective amount of the active agent that is administered to a particular subject in a particular instance will not always be effective in treating the conditions described herein, even though such dosage is deemed to be a therapeutically effective amount by those of skill in the art.

As used herein, "particulate" refers to a state of matter which is characterized by the presence of discrete particles, pellets, beads or granules irrespective of their size, shape or morphology.

As used herein, "multiparticulate" means a plurality of discrete, or aggregated, particles, pellets, beads, granules or mixture thereof irrespective of their size, shape or morphology. A composition comprising or consisting of a multiparticulate is described herein as a "multiparticulate composition".

As used herein, "nanoparticulate" refers to a multiparticulate in which the effective average particle size of the particles therein is less than about 2000 nm (2 microns) in diameter. A composition comprising a nanoparticulate is described herein as a "nanoparticulate composition."

As used herein, "effective average particle size" to describe a multiparticulate (e.g., a nanoparticulate) means that at least 50% of the particles thereof are of a specified size. [Or, that 50% of the particles are not above the specified size.] Correspondingly, "effective average particle size of less than about 2000 nm in diameter" means that at least 50% of the particles therein are less than about 2000 nm in diameter.

As used herein, "D50" refers to the particle size below which 50% of the particles in a multiparticulate fall. Similarly, "D90" refers to the particle size below which 90% of the particles in a multiparticulate fall.

As used herein with reference to stable particles, "stable" refers to, but is not limited to, one or more of the following parameters: (1) the particles do not appreciably flocculate or agglomerate due to interparticle attractive forces or otherwise significantly increase in particle size over time; (2) the physical structure of the particles is not altered over time, such as by conversion from an amorphous phase to a crystalline phase; (3) the particles are chemically stable; and/or (4) where the active ingredient has not been subject to a heating step at or above the melting point of the particles in the preparation of the nanoparticles of the present invention.

As used herein, "poorly water soluble drug" refers to a drug that has a solubility in water of less than about 30 mg/ml, less than about 20 mg/ml, less than about 10 mg/ml, or less than about 1 mg/ml, e.g. at room temperature.

As used herein, "modified release" includes a release which is not immediate and includes controlled release, extended release, sustained release and delayed release.

As used herein, "time delay" refers to the period of time between the administration of a dosage form comprising the composition of the invention and the release of the active ingredient from a particular component thereof.

As used herein, "lag time" refers to the time between the release of the active ingredient from one component of the composition and the release of the active ingredient from another component of the composition.

As used herein, "erodable" refers to formulations which may be worn away, diminished, or deteriorated by the action of substances within the body.

As used herein, "diffusion controlled" refers to formulations which may spread as the result of their spontaneous movement, for example, from a region of higher to one of lower concentration.

As used herein, "osmotic controlled" refers to formulations which may spread as the result of their movement through a semi-permeable membrane into a solution of higher concentration that tends to equalize the concentrations of the formulation on the two sides of the membrane.

As used herein, "cefdinir" refers to either a single substantially optically pure enantiomer of cefdinir or to a mixture, racemic or otherwise, of enantiomers of cefdinir.

The cephalosporin compositions herein are useful in the treatment of bacterial infection, including but not limited to, bronchitis, middle ear infections, pharyngitis, tonsillitis, pneumonia, sinus infections, and skin infections. The compositions of the invention are useful against a broad spectrum of Gram-positive and Gram-negative bacteria including but not limited to, *Staphylococcus epidermis, Staphylococcus aureus, Streptococcus algalctiae, Streptococcus pneumoniae, Streptococcus pyogenes, Moraxella catarrhalis, Escherichia coli, Citrobacter diversus, Klebsiella pneumoniae, Haemophilus influenzae, Haemophilus parainfluenzae, Neisseria gonorrhoeae, Propionobacterium acnes*, *Proteus mirabilis, Peptostreptococcus* sp., and *Providencia* sp.

### I. Nanoparticulate Compositions Comprising Cephalosporin

The present invention provides a nanoparticulate composition comprising particles which comprise: (A) a cephalosporin, for example cefdinir or a salt, derivative, prodrug, or polymorph thereof; and (B) at least one surface stabilizer. Nanoparticulate compositions were first described in U.S. Patent No. 5,145,684. Nanoparticulate active agent compositions are described also in, for example, U.S. Patent Nos. 5,298,262; 5,302,401; 5,318,767; 5,326,552; 5,328,404; 5,336,507; 5,340,564; 5,346,702; 5,349,957; 5,352,459; 5,399,363; 5,494,683; 5,401,492; 5,429,824; 5,447,710; 5,451,393; 5,466,440; 5,470,583; 5,472,683; 5,500,204; 5,518,738; 5,521,218; 5,525,328; 5,543,133; 5,552,160; 5,565,188; 5,569,448; 5,571,536; 5,573,749; 5,573,750; 5,573,783; 5,580,579; 5,585,108; 5,587,143; 5,591,456; 5,593,657; 5,622,938; 5,628,981; 5,643,552; 5,718,388; 5,718,919; 5,747,001; 5,834,025; 6,045,829; 6,068,858; 6,153,225; 6,165,506; 6,221,400; 6,264,922; 6,267,989; 6,270,806; 6,316,029; 6,375,986; 6,428,814; 6,431,478; 6,432,381; 6,582,285; 6,592,903; 6,656,504; 6,742,734; 6,745,962; 6,811,767; 6,908,626; 6,969,529; 6,976,647; and 6,991,191; and U.S. Patent Publication Nos. 20020012675; 20050276974; 20050238725; 20050233001; 20050147664; 20050063913; 20050042177; 20050031691; 20050019412; 20050004049; 20040258758; 20040258757; 20040229038; 20040208833; 20040195413;20040156895;20040156872;20040141925;20040115134;20040105889; 20040105778; 20040101566; 20040057905; 20040033267; 20040033202; 20040018242; 20040015134; 20030232796; 20030215502; 20030185869; 20030181411; 20030137067; 20030108616; 20030095928; 20030087308; 20030023203; 20020179758; 20020012675; and 20010053664. Amorphous small particle compositions are described, for example, in U.S. Patent Nos. 4,783,484; 4,826,689; 4,997,454; 5,741,522; 5,776,496.

As stated above, the effective average particle size of the particles in the nanoparticulate composition aspect herein, and preferably in other aspects, is less than about 2000 nm (i.e., 2 microns) in diameter. In embodiments of the present invention, the effective average particle size may be, for example, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm in diameter, as measured by light-scattering methods, microscopy, or other appropriate methods.

The nanoparticulate particles may exist in a crystalline phase, an amorphous phase, a semi-crystalline phase, a semi amorphous phase, or a mixture thereof.

In addition to allowing for a smaller solid dosage form size, we find that nanoparticulate compositions disclosed herein exhibit increased bioavailability, and require smaller doses of the cephalosporin, for example cefdinir or a salt, derivative, prodrug, or polymorph thereof, as compared to prior conventional, non-nanoparticulate compositions which comprise cephalosporin. In one embodiment of the invention, the nanoparticulate composition of the present invention has a bioavailability that is about 50% greater than cephalosporin, for example cefdinir or a salt, derivative, prodrug, or polymorph thereof, administered in a conventional dosage form. In other embodiments, the nanoparticulate composition of the present invention has a bioavailability that is about 40% greater, about 30% greater, about 20% or about 10% greater than cephalosporin, for example cefdinir or a salt, derivative, prodrug, or polymorph thereof, administered in a conventional dosage form.

The nanoparticulate composition may also have a desirable pharmacokinetic profile as measured following the initial dosage thereof to a mammalian subject. The desirable pharmacokinetic profile of the composition includes, but is not limited to: (1) a Cₘₐₓ for cephalosporin, for example cefdinir or a salt, derivative, prodrug, or polymorph thereof, when assayed in the plasma of a mammalian subject following administration that is preferably greater than the Cₘₐₓ for the same cephalosporin delivered at the same dosage by a non-nanoparticulate composition; and/or (2) an AUC for cephalosporin, for example cefdinir or a salt, derivative, prodrug, or polymorph thereof, when assayed in the plasma of a mammalian subject following administration that is preferably greater than the AUC for the same cephalosporin delivered at the same dosage by a non-nanoparticulate composition; and/or (3) a Tₘₐₓ for cephalosporin, for example cefdinir or a salt, derivative, prodrug, or polymorph thereof, when assayed in the plasma of a mammalian subject following administration that is preferably less than the Tₘₐₓ for the same cephalosporin delivered at the same dosage by a non-nanoparticulate composition.

In an embodiment of the present invention, a nanoparticulate composition of the present invention exhibits, for example, a Tₘₐₓ for cephalosporin, for example cefdinir or a salt, derivative, prodrug, or polymorph thereof, contained therein which is not greater than about 90% of the Tₘₐₓ for the same cephalosporin delivered at the same dosage by a non-nanoparticulate composition. In other embodiments of the present invention, the nanoparticulate composition of the present invention may exhibit, for example, a Tₘₐₓ for cephalosporin, for example cefdinir or a salt, derivative, prodrug, or polymorph thereof, contained therein which is not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ for the same cephalosporin delivered at the same dosage by a non-nanoparticulate composition. In one embodiment of the invention, the Tₘₐₓ of cephalosporin, when assayed in the plasma of the mammalian subject, is less than about 6 to about 8 hours after administration. In other embodiments of the invention, the Tₘₐₓ of cephalosporin is less than about 6 hours, less than about 5 hours, less than about 4 hours, less than about 3 hours, less than about 2 hours, less than about 1 hour, or less than about 30 minutes after administration.

In an embodiment of the present invention, a nanoparticulate composition of the present invention exhibits, for example, a Cₘₐₓ for cephalosporin, for example cefdinir or a salt, derivative, prodrug, or polymorph thereof, contained therein which is at least about 50% of the Cₘₐₓ for the same cephalosporin delivered at the same dosage by a non-nanoparticulate composition. In other embodiments of the present invention, the nanoparticulate composition of the present invention may exhibit, for example, a Cₘₐₓ for cephalosporin, for example cefdinir or a salt, derivative, prodrug, or polymorph thereof, contained therein which is at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ for the same cephalosporin delivered at the same dosage by a non-nanoparticulate composition.

In an embodiment of the present invention, a nanoparticulate composition of the present invention exhibits, for example, an AUC for cephalosporin, for example cefdinir or a salt, derivative, prodrug, or polymorph thereof, contained therein which is at least about 25% greater than the AUC for the same cephalosporin delivered at the same dosage by a non-nanoparticulate composition. In other embodiments of the present invention, the nanoparticulate composition of the present invention may exhibit, for example, an AUC for cephalosporin, for example cefdinir or a salt, derivative, prodrug, or polymorph thereof, contained therein which is at least about 50%, at least about 75%, at least about 100%, at least about 125%, at least about 150%, at least about 175%, at least about 200%, at least about 225%, at least about 250%, at least about 275%, at least about 300%, at least about 350%; at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC for the same cephalosporin delivered at the same dosage by a non-nanoparticulate composition.

The invention encompasses a nanoparticulate composition wherein the pharmacokinetic profile of cephalosporin, for example cefdinir or a salt, derivative, prodrug, or polymorph thereof, following administration is not substantially affected by the fed or fasted state of a subject ingesting the composition. This means that there is no substantial difference in the quantity of cephalosporin absorbed or the rate of absorption when the nanoparticulate composition is administered in the fed versus the fasted state. In conventional cephalosporin formulations, *i.e*., OMNICEF® and CEFZON®, the absorption of cephalosporin is increased when administered with food. This difference in absorption observed with conventional cephalosporin formulations is undesirable. We find that embodiments of the present compositions can overcome this problem.

Benefits of a dosage form which substantially eliminates the effect of food include an increase in subject convenience, thereby increasing subject compliance, as the subject does not need to ensure that they are taking a dose either with or without food. This is significant as, with poor subject compliance, an increase in the medical condition for which the cephalosporin is being prescribed may be observed.

The invention encompasses also a nanoparticulate composition comprising cephalosporin, for example cefdinir or a salt, derivative, prodrug, or polymorph thereof, in which administration of the composition to a subject in a fasted state is bioequivalent to administration of the composition to a subject in a fed state.

The difference in absorption of the composition of the invention, when administered in the fed versus the fasted state, preferably is less than about 100%, less than bout 95%, less than about 90%, less than about 85%, less than about 80%, less than about 75%, less than about 70%, less than about 65%, less than about 60%, less than about 55%, less than about 50%, less than about 45%, less than about 40%, less than about 35%, less than about 30%, less than about 25%, less than about 20%, less than about 15%, less than about 10%, less than about 5%, or less than about 3%.

In one embodiment of the invention, the invention encompasses a composition comprising cephalosporin, for example cefdinir or a salt, derivative, prodrug, or polymorph thereof, wherein the administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% CI for AUC must be between 0.80 to 1.25 and the 90% CI for Cₘₐₓ must between 0.70 to 1.43.

The nanoparticulate composition of the invention is proposed to have an unexpectedly dramatic dissolution profile. Rapid dissolution of cephalosporin, for example cefdinir or a salt, derivative, prodrug, or polymorph thereof, is preferable, as faster dissolution generally leads to faster onset of action and greater bioavailability. To improve the dissolution profile and bioavailability of the cephalosporin, it would be useful to increase the drug's dissolution so that it could attain a level close to 100%.

The compositions of the invention preferably have a dissolution profile in which within about 5 minutes at least about 20% of the cephalosporin, for example cefdinir or a salt, derivative, prodrug, or polymorph thereof, is dissolved. In other embodiments of the invention, at least about 30% or at least about 40% of the cephalosporin, for example cefdinir or a salt, derivative, prodrug, or polymorph thereof, is dissolved within about 5 minutes. In yet other embodiments of the invention, preferably at least about 40%, at least about 50%, at least about 60%, at least about 70%, or at least about 80% of the cephalosporin, for example cefdinir or a salt, derivative, prodrug, or polymorph thereof, is dissolved within about 10 minutes. Finally, in another embodiment of the invention, preferably at least about 70%, at least about 80%, at least about 90%, or at least about 100% of the cephalosporin, for example cefdinir or a salt, derivative, prodrug, or polymorph thereof, is dissolved within about 20 minutes.

Dissolution is preferably measured in a medium which is discriminating. Such a dissolution medium will produce two very different dissolution curves for two products having very different dissolution profiles in gastric juices;.*i.e*., the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

An additional feature of nanoparticulate compositions herein is that particles thereof redisperse so that the particles have an effective average particle size of less than about 2000 nm in diameter. This is significant because, if the particles did not redisperse so that they have an effective average particle size of less than about 2000 nm in diameter, the composition may lose the benefits afforded by formulating the cephalosporin therein into a nanoparticulate form. This is because nanoparticulate compositions benefit from the small size of the particles comprising the cephalosporin. If the particles do not redisperse into small particle sizes upon administration, then "clumps" or agglomerated particles are formed, owing to the extremely high surface free energy of the nanoparticulate system and the thermodynamic driving force to achieve an overall reduction in free energy. With the formation of such agglomerated particles, the bioavailability of the dosage form may fall well below that observed with the liquid dispersion form of the nanoparticulate composition.

In other embodiments of the invention, the redispersed particles of the invention (redispersed in water, a biorelevant media, or any other suitable liquid media) have an effective average particle size of less than about less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm in diameter, as measured by light-scattering methods, microscopy, or other appropriate methods. Such methods suitable for measuring effective average particle size are known to a person of ordinary skill in the art.

Redispersibility can be tested using any suitable means known in the art. See *e.g*.; the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

Nanoparticulate compositions herein are found to exhibit dramatic redispersion of the particles upon administration to a mammal, such as a human or animal, as demonstrated by reconstitution/redispersion in a biorelevant aqueous media, such that the effective average particle size of the redispersed particles is less than about 2000 nm. Such biorelevant aqueous media can be any aqueous media that exhibits the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. *See e.g.,* Lindahl et al., "Characterization of Fluids from the Stomach and Proximal Jejunum in Men and Women," Pharm. Res., 14 (4): 497-502 (1997). It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs *(i.e.,* weak acids and corresponding salts of that acid), monoprotic and polyprotic electrolytes, etc.

Representative electrolyte solutions can be, but are not limited to, HCl solutions, ranging in concentration from about 0.001 to about 0.1 N, and NaCl solutions, ranging in concentration from about 0.001 to about 0.1 M, and mixtures thereof. For example, electrolyte solutions can be, but are not limited to, about 0.1 N HCl or less, about 0.01 N HCl or less, about 0.001 N HCl or less, about 0.1 M NaCl or less, about 0.01 M NaCl or less, about 0.001 M NaCl or less, and mixtures thereof. Of these electrolyte solutions, 0.01 M HCl and/or 0.1 M Nacl, are most representative of fasted human physiological conditions, owing to the pH and ionic strength conditions of the proximal gastrointestinal tract.

Electrolyte concentrations of 0.001 N HCI, 0.01 N HCI, and 0.1 N HCI correspond to pH 3, pH 2, and pH 1, respectively. Thus, a 0.01 N HCl solution simulates typical acidic conditions found in the stomach. A solution of 0.1 M NaCI provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

As stated above, the composition comprises also at least one surface stabilizer. The surface stabilizer can be adsorbed on or associated with the surface of the cephalosporin-containing particles. Preferably, the surface stabilizer adheres on, or associates with, the surface of the particles, but does not react chemically with the particles or with other surface stabilizer molecules. Individually adsorbed molecules of the surface stabilizer are essentially free of intermolecular cross-linkages.

The relative amounts of the cephalosporin, for example cefdinir or a salt, derivative, prodrug, or polymorph thereof, and surface stabilizer present in the composition of the present invention can vary widely. The optional amount of the individual components can depend, upon, among other things, the particular drug selected, the hydrophilic-lipophilic balance (HLB), melting point, and the surface tension of water solutions of the stabilizer. The concentration of the cephalosporin, for example cefdinir or a salt, derivative, prodrug, or polymorph thereof, can vary from about 99.5% to about 0.00 1 %, from about 95% to about 0.1 %, or from about 90% to about 0.5%, by weight, based on the total combined weight of the cephalosporin and surface stabilizer(s), not including other excipients. The concentration of the surface stabilizer(s) can vary from about 0.5% to about 99.999%, from about 5.0% to about 99.9%, or from about 10% to about 99.5%, by weight, based on the total combined dry weight of the cepahlosporin and surface stabilizer(s), not including other excipients.

The choice of a surface stabilizer(s) for the cephalosporin is non-trivial and required extensive experimentation to realize a desirable formulation. Accordingly, an aspect of the present findings is the surprising discovery that nanoparticulate compositions comprising cephalosporin, in particular cefdinir or a salt, derivative, prodrug, or polymorph thereof, can be made.

Combinations of more than one surface stabilizer can be used. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, anionic, cationic, ionic, and zwitterionic surfactants.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g*., the commercially available Tweens® such as e.g., Tween 20® and Tween 80® (ICI Speciality Chemicals)); polyethylene glycols (e.g., Carbowaxs 3550® and 934® (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronics F68® and F108®, which are block copolymers of ethylene oxide and propylene oxide); poloxamines (e.g., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation), Tritons X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-lOG® or Surfactant 10-G® (Olin Chemicals, Stamford, CT); Crodestas SL-40® (Croda, Inc.); and SA9OHCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyldimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quaternized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁ -R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quatemium-5), dodecyl dimethyl ethylbenzyl ammonium chloride(Quatemium-14), Quatemium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000).

The compositions of the invention can comprise, in addition to a cephalosporin, one or more other compounds useful in treating bacterial infections. The composition may also be administered in conjunction with such a compound. Examples of such compounds include antibiotics.

The composition of the present invention may comprise also one or more binding agents, filling agents, diluents, lubricating agents, emulsifying and suspending agents, sweeteners, flavoring agents, preservatives, buffers, wetting agents, disintegrants, effervescent agents, perfuming agents, and other excipients. Such excipients are known in the art. In addition, prevention of the growth of microorganisms can be ensured by the addition of various antibacterial and antifungal agents, such as parabens, chlorobutanol, phenol, sorbic acid, and the like. For use in injectable formulations, the composition may comprise also isotonic agents, such as sugars, sodium chloride, and the like and agents for use in delaying the absorption of the injectable pharmaceutical form, such as aluminum monostearate and gelatin.

Examples of filling agents are lactose monohydrate, lactose anhydrous, and various starches; examples of binding agents are various celluloses and cross-linked polyvinylpyrrolidone, microcrystalline cellulose, such as Avicel® PH101 and Avicel® PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil® 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet® (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel® PH101 and Avicel® PH 102; lactose such as lactose monohydrate, lactose anhydrous, and Pharmatose® DCL21; dibasic calcium phosphate such as Emcompress®; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

The composition of the present invention may comprise also a carrier, adjuvant, or a vehicle (hereafter, collectively, "carriers").

The nanoparticulate compositions can be made using, for example, milling, homogenization, precipitation, freezing, or template emulsion techniques. Exemplary methods of making nanoparticulate compositions are described in the '684 patent. Methods of making nanoparticulate compositions are described also in U.S. Patent Nos. 5,518,187; 5,718,388; 5,862,999; 5,665,331; 5,662,883; 5,560,932; 5,543,133; 5,534,270; 5,510,118; and 5,470,583.

In one method, particles comprising cephalosporin are dispersed in a liquid dispersion medium in which the cephalosporin is poorly soluble. Mechanical means are then used in the presence of grinding media to reduce the particle size to the desired effective average particle size. The dispersion medium can be, for example, water, safflower oil, ethanol, t-butanol, glycerin, polyethylene glycol (PEG), hexane, or glycol. A preferred dispersion medium is water. The particles can be reduced in size in the presence of at least one surface stabilizer. The particles comprising cephalosporin can be contacted with one or more surface stabilizers after attrition. Other compounds, such as a diluent, can be added to the composition during the size reduction process. Dispersions can be manufactured continuously or in a batch mode. One skilled in the art would understand that it may be the case that, following milling, not all particles may be reduced to the desired size. In such an event, the particles of the desired size may be separated and used in the practice of the present invention.

Another method of forming the desired nanoparticulate composition is by microprecipitation. This is a method of preparing stable dispersions of poorly soluble cephalosporin in the presence of surface stabilizer(s) and one or more colloid stability-enhancing surface active agents free of any trace toxic solvents or solubilized heavy metal impurities. Such a method comprises, for example: (1) dissolving the cephalosporin, for example cefdinir or a salt, derivative, prodrug, or polymorph thereof, in a suitable solvent; (2) adding the formulation from step (1) to a solution comprising at least one surface stabilizer, and (3) precipitating the formulation from step (2) using an appropriate non-solvent. The method can be followed by removal of any formed salt, if present, by dialysis or diafiltration and concentration of the dispersion by conventional means.

A nanoparticulate composition may be formed also by homogenization. Exemplary homogenization methods are described in U.S. Patent No. 5,510,118, for "Process of Preparing Therapeutic Compositions Containing Nanoparticles." Such a method comprises dispersing particles comprising cephalosporin, for example cefdinir or a salt, derivative, prodrug, or polymorph thereof, in a liquid dispersion medium, followed by subjecting the dispersion to homogenization to reduce the particle size to the desired effective average particle size. The particles can be reduced in size in the presence of at least one surface stabilizer. The particles can be contacted with one or more surface stabilizers either before or after attrition. Other compounds, such as a diluent, can be added to the composition before, during, or after the size reduction process. Dispersions can be manufactured continuously or in a batch mode.

Another method of forming the desired nanoparticulate composition is by spray freezing into liquid (SFL). This technology comprises injecting an organic or organoaqueous solution of cephalosporin, for example cefdinir or a salt, derivative, prodrug, or polymorph thereof, and surface stabilizer(s) into a cryogenic liquid, such as liquid nitrogen. The droplets of the drug-containing solution freeze at a rate sufficient to minimize crystallization and particle growth, thus formulating nano-structured particles. Depending on the choice of solvent system and processing conditions, the particles can have varying particle morphology. In the isolation step, the nitrogen and solvent are removed under conditions that avoid agglomeration or ripening of the particles.

As a complementary technology to SFL, ultra rapid freezing (URF) may also be used to create equivalent nanostructured particles with greatly enhanced surface area. URF comprises taking a water-miscible, anhydrous, organic, or organoaqueous solution of cephalosporin, for example cefdinir or a salt, derivative, prodrug, or polymorph thereof, and surface stabilizer(s) and applying it onto a cryogenic substrate. The solvent is then removed by means such as lyophilization or atmospheric freeze-drying with the resulting nanostructured particles remaining.

Another method of forming the desired nanoparticulate composition is by template emulsion. Template emulsion creates nano-structured particles with controlled particle size distribution and rapid dissolution performance. The method comprises preparing an oil-in-water emulsion and then swelling it with a non-aqueous solution comprising cephalosporin, for example cefdinir or a salt, derivative, prodrug, or polymorph thereof, and surface stabilizer(s). The size distribution of the particles is a direct result of the size of the emulsion droplets prior to loading of the emulsion with the drug. The particle size can be controlled and optimized in this process. Furthermore, through selected use of solvents and stabilizers, emulsion stability is achieved with no or suppressed Ostwald ripening. Subsequently, the solvent and water are removed, and the stabilized nano-structured particles are recovered. Various particle morphologies can be achieved by appropriate control of processing conditions.

The invention also provides a method comprising the administration of an effective amount of a nanoparticulate composition comprising cephalosporin, for example cefdinir or a salt, derivative, prodrug, or polymorph thereof.

The composition of the present invention can be formulated for administration parentally (*e.g*., intravenous, intramuscular, or subcutaneous), orally (*e.g*., in solid, liquid, or aerosol form, vaginal), nasally, rectally, ocularly, locally (*e.g*., in powder, ointment, or drop form), buccally, intracisternally, intraperitoneally, or topically, and the like.

The nanoparticulate composition can be utilized in solid or liquid dosage formulations, such as liquid dispersions, gels, aerosols, ointments, depots, creams, controlled release formulations, fast melt formulations, lyophilized formulations, tablets, capsules, delayed release formulations, extended release formulations, pulsatile release formulations, mixed immediate release and controlled release formulations, etc.

Compositions suitable for parenteral injection may comprise physiologically acceptable sterile aqueous or non-aqueous solutions, dispersions, suspensions or emulsions, and sterile powders for reconstitution into sterile injectable solutions or dispersions. Examples of suitable aqueous and non-aqueous carriers, diluents, solvents, or vehicles including water, ethanol, polyols (propyleneglycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

Solid dosage forms for oral administration include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to cephalosporin, for example cefdinir or a salt, derivative, prodrug, or polymorph thereof, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

One of ordinary skill will appreciate that a therapeutically effective amount of cephalosporin, for example cefdinir or a salt, derivative, prodrug, or polymorph thereof, can be determined empirically. Actual dosage levels of cephalosporin in the nanoparticulate compositions of the invention may be varied to obtain an amount of the drug that is effective to obtain a desired therapeutic response for a particular composition and method of administration. The selected dosage level therefore depends upon the desired therapeutic effect, the route of administration, the potency of the administered cephalosporin, the desired duration of treatment, and other factors.

Dosage unit compositions may contain such amounts of cephalosporin or such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the cephalosporin; the duration of the treatment; active compound used in combination or coincidental with cephalosporin; and like factors well known in the medical arts.

### II. Controlled Release Compositions Comprising Ceophalosporin

The effectiveness of pharmaceutical compounds in the prevention and treatment of disease states depends on a variety of factors including the rate and duration of delivery of the compound from the dosage form to the patient. The combination of delivery rate and duration exhibited by a given dosage form in a patient can be described as its *in vivo* release profile and, depending on the pharmaceutical compound administered, will be associated with a concentration and duration of the pharmaceutical compound in the blood plasma, referred to as a plasma profile. As pharmaceutical compounds vary in their pharmacokinetic properties such as bioavailability, and rates of absorption and elimination, the release profile and the resultant plasma profile become important elements to consider in designing effective therapies.

The release profiles of dosage forms may exhibit different rates and durations of release and may be continuous or pulsatile. Continuous release profiles include release profiles in which a quantity of one or more pharmaceutical compounds is released continuously throughout the dosing interval at either a constant or variable rate. Pulsatile release profiles include release profiles in which at least two discrete quantities of one or more pharmaceutical compounds are released at different rates and/or over different time frames. For any given pharmaceutical compound or combination of such compounds, the release profile for a given dosage form gives rise to an associated plasma profile in a patient. When two or more components of a dosage form have different release profiles, the release profile of the dosage form as a whole is a combination of the individual release profiles and may be described generally as "multimodal." The release profile of a two-component dosage form in which each component has a different release profile may described as "bimodal," and the release profile of a three-component dosage form in which each component has a different release profile may described as "trimodal."

Similar to the variables applicable to the release profile, the associated plasma profile in a patient may exhibit constant or variable blood plasma concentration levels of the pharmaceutical compounds over the duration of action and may be continuous or pulsatile. Continuous plasma profiles include plasma profiles of all rates and duration which exhibit a single plasma concentration maximum. Pulsatile plasma profiles include plasma profiles in which at least two higher blood plasma concentration levels of pharmaceutical compound are separated by a lower blood plasma concentration level and may be described generally as "multimodal." Pulsatile plasma profiles exhibiting two peaks may be described as "bimodal" and plasma profiles exhibiting three peaks may be described as "trimodal." Depending on, at least in part, the pharmacokinetics of the pharmaceutical compounds included in the dosage form as well as the release profiles of the individual components of the dosage form, a multimodal release profile may result in either a continuous or a pulsatile plasma profile upon administration to a patient.

In one aspect, and in preferred embodiments of other aspects, we provide a multiparticulate modified release composition which delivers cephalosporin, for example cefdinir or a salt, derivative, prodrug, or polymorph thereof, or nanoparticles containing the same, in a pulsatile manner. The nanoparticles are of the type described above and comprise also at least one surface stabilizer.

In still another aspect, the present invention provides a multiparticulate modified release composition which delivers cephalosporin, for example cefdinir or a salt, derivative, prodrug, or polymorph thereof, or nanoparticles containing the same, in a continuous manner. The nanoparticles are of the type described above and comprise also at least one surface stabilizer.

In yet another aspect, the present invention provides a multiparticulate modified release composition in which a first portion of cephalosporin, for example cefdinir or a salt, derivative, prodrug, or polymorph thereof, or nanoparticles containing the same, is released immediately upon administration and one or more subsequent portions of cephalosporin, for example cefdinir or a salt, derivative, prodrug, or polymorph thereof, or nanoparticles containing the same, are released after an initial time delay.

In yet another aspect, the present invention provides solid oral dosage forms for once-daily or twice-daily administration comprising a multiparticulate modified release composition as disclosed herein.

In still another embodiment, the present invention provides a method for the prevention and/or treatment of a bacterial infection comprising the administration of a composition of the present invention.

In an embodiment, the present invention provides a multiparticulate modified release composition in which the particles forming the multiparticulate are nanoparticulate particles of the type described above. The nanoparticulate particles may, as desired, contain a modified release coating and/or a modified release matrix material.

According to a further aspect herein, there is provided a pharmaceutical composition having a first component comprising active ingredient-containing particles, and at least one subsequent component comprising active ingredient-containing particles, each subsequent component having a rate and/or duration of release different from the first component wherein at least one of said components comprises particles containing cephalosporin, for example cefdinir or a salt, derivative, prodrug, or polymorph thereof. In an embodiment of this, cephalosporin-containing particles that form the multiparticulate may themselves contain nanoparticulate particles of the type described above which comprise cephalosporin, for example cefdinir or a salt, derivative, prodrug, or polymorph thereof, and also at least one surface stabilizer. In another embodiment of the invention, nanoparticulate particles of the type described above which comprise cephalosporin, for example cefdinir or a salt, derivative, prodrug, or polymorph thereof, and also at least one surface stabilizer themselves are the drug-containing particles of the multiparticulate. The drug-containing particles may be coated with a modified release coating. Alternatively or additionally, the drug-containing particles may comprise a modified release matrix material. Following oral delivery, the composition delivers cephalosporin, for example cefdinir or a salt, derivative, prodrug, or polymorph thereof, or nanoparticles containing the same, in a pulsatile manner. In one embodiment, the first component provides an immediate release of cephalosporin, for example cefdinir or a salt, derivative, prodrug, or polymorph thereof, or nanoparticles containing the same, and the one or more subsequent components provide a modified release of cephalosporin, for example cefdinir or a salt, derivative, prodrug, or polymorph thereof, or nanoparticles containing the same. In such embodiments, the immediate release component serves to hasten the onset of action by minimizing the time from administration to a therapeutically effective plasma concentration level, and the one or more subsequent components serve to minimize the variation in plasma concentration levels and/or maintain a therapeutically effective plasma concentration throughout the dosing interval.

The modified release coating and/or the modified release matrix material cause a lag time between the release of the active ingredient from the first population of active ingredient-containing particles and the release of the active ingredient from subsequent populations of active ingredient-containing particles. Where more than one population of active ingredient-containing particles provide a modified release, the modified release coating and/or the modified release matrix material causes a lag time between the release of the active ingredient from the different populations of active ingredient-containing particles. The duration of these lag times may be varied by altering the composition and/or the amount of the modified release coating and/or altering the composition and/or amount of modified release matrix material utilized. Thus, the duration of the lag time can be designed to mimic a desired plasma profile.

Because the plasma profile produced by the modified release composition upon administration is substantially similar to the plasma profile produced by the administration of two or more IR dosage forms given sequentially, the modified release composition of the present invention is particularly useful for administering cephalosporin, for example cefdinir, or a salt, derivative, prodrug, or polymorph thereof.

According to another aspect of the present invention, the composition can be designed to produce a plasma profile that minimizes or eliminates the variations in plasma concentration levels associated with the administration of two or more IR dosage forms given sequentially. In such embodiments, the composition may be provided with an immediate release component to hasten the onset of action by minimizing the time from administration to a therapeutically effective plasma concentration level, and at least one modified release component to maintain a therapeutically effective plasma concentration level throughout the dosing interval.

The active ingredients in each component may be the same or different. For example, the composition may comprise components comprising only cephalosporin, for example cefdinir or a salt, derivative, prodrug, or polymorph thereof, or nanoparticles containing the same, as the active ingredient. Alternatively, the composition may comprise a first component comprising cephalosporin, for example cefdinir or a salt, derivative, prodrug, or polymorph thereof, or nanoparticles containing the same, and at least one subsequent component comprising an active ingredient other than cephalosporin, or nanoparticles containing cephalosporin, suitable for co-administration with cephalosporin, or a first component containing an active ingredient other than cephalosporin, or nanoparticles containing cephalosporin, and at least one subsequent component comprising cephalosporin, for example cefdinir, or a salt, derivative, prodrug, or polymorph thereof, or nanoparticles containing the same. Indeed, two or more active ingredients may be incorporated into the same component when the active ingredients are compatible with each other. An active ingredient present in one component of the composition may be accompanied by, for example, an enhancer compound or a sensitizer compound in another component of the composition, in order to modify the bioavailability or therapeutic effect thereof.

As used herein, the term "enhancer" refers to a compound which is capable of enhancing the absorption and/or bioavailability of an active ingredient by promoting net transport across the GIT in an animal, such as a human. Enhancers include but are not limited to medium chain fatty acids; salts, esters, ethers and derivatives thereof, including glycerides and triglycerides; non-ionic surfactants such as those that can be prepared by reacting ethylene oxide with a fatty acid, a fatty alcohol, an alkylphenol or a sorbitan or glycerol fatty acid ester; cytochrome P450 inhibitors, P-glycoprotein inhibitors and the like; and mixtures of two or more of these agents.

In those embodiments in which more than one drug-containing component is present, the proportion of cephalosporin contained in each component may be the same or different depending on the desired dosing regime. The cephalosporin present in the first component and in subsequent components may be any amount sufficient to produce a therapeutically effective plasma concentration level. The cephalosporin, when applicable, may be present either in the form of one substantially optically pure stereoisomer or as a mixture, racemic or otherwise, of two or more stereoisomers. In one embodiment, the cephalosporin is present in the composition in an amount of from about 0.1 to about 500 mg. In another embodiment, the cephalosporin is present in the composition in an amount of from about 1 to about 100 mg. In yet another embodiment, the cephalosporin is present in the first component in an amount of from about 0.5 to about 60 mg. In still another embodiment, the cephalosporin is present in the first component in an amount of from about 2.5 to about 30 mg. If in subsequent components, the cephalosporin is present in amounts within similar ranges to those described for the first component.

The time release characteristics for the delivery of cephalosporin from each of the components may be varied by modifying the composition of each component, including modifying any of the excipients and/or coatings which may be present. In particular, the release of cephalosporin may be controlled by changing the composition and/or the amount of the modified release coating on the particles, if such a coating is present. If more than one modified release component is present, the modified release coating for each of these components may be the same or different. Similarly, when modified release is facilitated by the inclusion of a modified release matrix material, release of the active ingredient may be controlled by the choice and amount of modified release matrix material utilized. The modified release coating may be present, in each component, in any amount that is sufficient to yield the desired delay time for each particular component. The modified release coating may be preset, in each component, in any amount that is sufficient to yield the desired time lag between components.

The lag time and/or time delay for the release of cephalosporin from each component may also be varied by modifying the composition of each of the components, including modifying any excipients and coatings which may be present. For example, the first component may be an immediate release component wherein cephalosporin, for example cefdinir, or a salt, derivative, prodrug, or polymorph thereof, is released immediately upon administration. Alternatively, the first component may be, for example, a time-delayed immediate release component in which cephalosporin, for example cefdinir, or a salt, derivative, prodrug, or polymorph thereof, is released substantially in its entirety immediately after a time delay. The second and subsequent component may be, for example, a time-delayed immediate release component as just described or, alternatively, a time-delayed sustained release or extended release component in which cephalosporin, for example cefdinir, or a salt, derivative, prodrug, or polymorph thereof, is released in a controlled fashion over an extended period of time.

As will be appreciated by those skilled in the art, the exact nature of the plasma concentration curve will be influenced by the combination of all of these factors just described. In particular, the lag time between the delivery (and thus also the onset of action) of cephalosporin, for example cefdinir, or a salt, derivative, prodrug, or polymorph thereof, in each component containing cephalosporin may be controlled by varying the composition and coating (if present) of each of the components. Thus by variation of the composition of each component (including the amount and nature of the active ingredient(s)) and by variation of the lag time, numerous release and plasma profiles may be obtained. Depending on the duration of the lag time between the release of cephalosporin from each component and the nature of the release of cephalosporin from each component (i.e. immediate release, sustained release etc.), the plasma profile may be continuous (i.e., having a single maximum) or pulsatile in which the peaks in the plasma profile may be well separated and clearly defined (e.g. when the lag time is long) or superimposed to a degree (e.g. when the lag time is short).

The plasma profile produced from the administration of a single dosage unit comprising the composition of the present invention is advantageous when it is desirable to deliver two or more pulses of active ingredient without the need for administration of two or more dosage units.

Any coating material which modifies the release of cephalosporin, for example cefdinir, or a salt, derivative, prodrug, or polymorph thereof, in the desired manner may be used. In particular, coating materials suitable for use in the practice of the present invention include but are not limited to polymer coating materials, such as cellulose acetate phthalate, cellulose acetate trimaletate, hydroxy propyl methylcellulose phthalate, polyvinyl acetate phthalate, ammonio methacrylate copolymers such as those sold under the trademark Eudragit® RS and RL, poly acrylic acid and poly acrylate and methacrylate copolymers such as those sold under the trademark Eudragit® S and L, polyvinyl acetaldiethylamino acetate, hydroxypropyl methylcellulose acetate succinate, shellac; hydrogels and gel-forming materials, such as carboxyvinyl polymers, sodium alginate, sodium carmellose, calcium carmellose, sodium carboxymethyl starch, polyvinyl alcohol, hydroxyethyl cellulose, methyl cellulose, gelatin, starch, and cellulose based cross-linked polymers--in which the degree of crosslinking is low so as to facilitate adsorption of water and expansion of the polymer matrix, hydoxypropyl cellulose, hydroxypropyl methylcellulose, polyvinylpyrrolidone, crosslinked starch, microcrystalline cellulose, chitin, aminoacryl-methacrylate copolymer (Eudragit® RS-PM, Rohm & Haas), pullulan, collagen, casein, agar, gum arabic, sodium carboxymethyl cellulose, (swellable hydrophilic polymers) poly(hydroxyalkyl methacrylate) (mol. wt. ~5k-5,000k), polyvinylpyrrolidone (mol. wt. ~10k-360k), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (mol. wt. ~30k-300k), polysaccharides such as agar, acacia, karaya, tragacanth, algins and guar, polyacrylamides, Polyox® polyethylene oxides (mol. wt. ~100k-5,000k), AquaKeep® acrylate polymers, diesters of polyglucan, crosslinked polyvinyl alcohol and poly N-vinyl-2-pyrrolidone, sodium starch glucolate (e.g. Explotab®; Edward Mandell C. Ltd.); hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, polyethylene oxides (e.g. Polyox®, Union Carbide), methyl ethyl cellulose, ethylhydroxy ethylcellulose, cellulose acetate, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers of methacrylic acid or methacrylic acid (e.g. Eudragit®, Rohm and Haas), other acrylic acid derivatives, sorbitan esters, natural gums, lecithins, pectin, alginates, ammonia alginate, sodium, calcium, potassium alginates, propylene glycol alginate, agar, and gums such as arabic, karaya, locust bean, tragacanth, carrageens, guar, xanthan, scleroglucan and mixtures and blends thereof. As will be appreciated by the person skilled in the art, excipients such as plasticisers, lubricants, solvents and the like may be added to the coating. Suitable plasticisers include for example acetylated monoglycerides; butyl phthalyl butyl glycolate; dibutyl tartrate; diethyl phthalate; dimethyl phthalate; ethyl phthalyl ethyl glycolate; glycerin; propylene glycol; triacetin; citrate; tripropioin; diacetin; dibutyl phthalate; acetyl monoglyceride; polyethylene glycols; castor oil; triethyl citrate; polyhydric alcohols, glycerol, acetate esters, gylcerol triacetate, acetyl triethyl citrate, dibenzyl phthalate, dihexyl phthalate, butyl octyl phthalate, diisononyl phthalate, butyl-octyl phthalate, dioctyl azelate, epoxidised tallate, triisoctyl trimellitate, diethylhexyl phthalate, di-n-octyl phthalate, di-i-octyl phthalate, di-i-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate.

When the modified release component comprises a modified release matrix material, any suitable modified release matrix material or suitable combination of modified release matrix materials may be used. Such materials are known to those skilled in the art. The term "modified release matrix material" as used herein includes hydrophilic polymers, hydrophobic polymers and mixtures thereof which are capable of modifying the release of cephalosporin, for example cefdinir, or a salt, derivative, prodrug, or polymorph thereof, dispersed therein in vitro or in vivo. Modified release matrix materials suitable for the practice of the present invention include but are not limited to microcrystalline cellulose, sodium carboxymethylcellulose, hydoxyalkylcelluloses such as hydroxypropylmethylcellulose and hydroxypropylcellulose, polyethylene oxide, alkylcelluloses such as methylcellulose and ethylcellulose, polyethylene glycol, polyvinylpyrrolidone, cellulose acteate, cellulose acetate butyrate, cellulose acteate phthalate, cellulose acteate trimellitate, polyvinylacetate phthalate, polyalkylmethacrylates, polyvinyl acetate and mixture thereof.

A modified release composition as proposed herein may be incorporated into any suitable dosage form which facilitates release of the active ingredient in a pulsatile manner. In one embodiment, the dosage form comprises a blend of different populations of active ingredient-containing particles which make up the immediate release and the modified release components, the blend being filled into suitable capsules, such as hard or soft gelatin capsules. Alternatively, the different individual populations of active ingredient-containing particles may be compressed (optionally with additional excipients) into mini-tablets which may be subsequently filled into capsules in the appropriate proportions. Another suitable dosage form is that of a multilayer tablet. In this instance the first component of the modified release composition may be compressed into one layer, with the subsequent component being subsequently added as a subsequent layer of the multilayer tablet. The populations of the particles making up the composition of the invention may further be included in rapidly dissolving dosage forms such as an effervescent dosage form or a fast-melt dosage form.

In one embodiment, the composition comprises at least two components containing cephalosporin, for example cefdinir, or a salt, derivative, prodrug, or polymorph thereof: a first component and one or more subsequent components. In such embodiment, the first component of the composition may exhibit a variety of release profiles including profiles in which substantially all of the cephalosporin contained in the first component is released rapidly upon administration of the dosage form, released rapidly but after a time delay (delayed release), or released slowly over time. In one such embodiment, the cephalosporin contained in the first component is released rapidly upon administration to a patient. As used herein, "released rapidly" includes release profiles in which at least about 80% of the active ingredient of a component is released within about an hour after administration, the term "delayed release" includes release profiles in which the active ingredient of a component is released (rapidly or slowly) after a time delay, and the terms "controlled release" and "extended release" include release profiles in which at least about 80% of the active ingredient contained in a component is released slowly.

The second component of such embodiment may also exhibit a variety of release profiles including an immediate release profile, a delayed release profile or a controlled release profile. In one such embodiment, the second component exhibits a delayed release profile in which cephalosporin is released after a time delay.

The plasma profile produced by the administration of dosage forms of the present invention which comprise an immediate release component comprising cephalosporin, or nanoparticles containing cephalosporin, and at least one modified release component comprising cephalosporin, or nanoparticles containing cephalosporin, can be substantially similar to the plasma profile produced by the administration of two or more IR dosage forms given sequentially, or to the plasma profile produced by the administration of separate IR and modified release dosage forms. Accordingly, the dosage forms of the present invention can be particularly useful for administering cephalosporin, for example cefdinir, or a salt, derivative, prodrug, or polymorph thereof, where the maintenance of pharmacokinetic parameters may be desired but is problematic.

In one embodiment, the composition and the solid oral dosage forms containing the composition release cephalosporin, for example cefdinir, or a salt, derivative, prodrug, or polymorph thereof, such that substantially all of the cephalosporin contained in the first component is released prior to release of cephalosporin from the at least one subsequent component. When the first component comprises an IR component, for example, it is preferable that release of the cephalosporin from the at least one subsequent component is delayed until substantially all cephalosporin in the IR component has been released. Release of cephalosporin from the at least one subsequent component may be delayed as detailed above by the use of a modified release coatings and/or a modified release matrix material.

When it is desirable to minimize patient tolerance by providing a dosage regime which facilitates wash-out of a first dose of cephalosporin from a patient's system, release of cephalosporin, for example cefdinir, or a salt, derivative, prodrug, or polymorph thereof, from subsequent components may be delayed until substantially all of the cepahlosporin contained in the first component has been released, and further delayed until at least a portion of the cephalosporin released from the first component has been cleared from the patient's system. In one embodiment, release of the cephalosporin, for example cefdinir, or a salt, derivative, prodrug, or polymorph thereof, from subsequent components of the composition is substantially, if not completely, delayed for a period of at least about two hours after administration of the composition. In another embodiment, the release of cephalosporin, for example cefdinir, or a salt, derivative, prodrug, or polymorph thereof, from subsequent components of the composition is substantially, if not completely, delayed for a period of at least about four hours after administration of the composition.

As described hereinbelow, the present invention also includes various types of modified release systems by which cephalosporin, for example cefdinir, or a salt, derivative, prodrug, or polymorph thereof, may be delivered in either a pulsatile or continuous manner. These systems include but are not limited to: films with cephalosporin, for example cefdinir, or a salt, derivative, prodrug, or polymorph thereof, or nanoparticles containing cephalosporin, for example cefdinir, or a salt, derivative, prodrug, or polymorph thereof, in a polymer matrix (monolithic devices); systems in which cephalosporin, for example cefdinir, or a salt, derivative, prodrug, or polymorph thereof, or nanoparticles containing the same, is contained by a polymer (reservoir devices); polymeric colloidal particles or microencapsulates (microparticles, microspheres or nanoparticles) in the form of reservoir and matrix devices; systems in which cephalosporin, for example cefdinir, or a salt, derivative, prodrug, or polymorph thereof, or nanoparticles containing the same, is contained by a polymer which contains a hydrophilic and/or leachable additive e.g., a second polymer, surfactant or plasticizer, etc. to give a porous device, or a device in which cephalosporin release may be osmotically controlled (both reservoir and matrix devices); enteric coatings (ionizable and dissolve at a suitable pH); (soluble) polymers with (covalently) attached pendent cephalosporin molecules and devices where release rate is controlled dynamically: e.g., the osmotic pump.

The delivery mechanism of the present invention can control the rate of release of cephalosporin, for example cefdinir, or a salt, derivative, prodrug, or polymorph thereof. While some mechanisms will release cephalosporin at a constant rate, others will vary as a function of time depending on factors such as changing concentration gradients or additive leaching leading to porosity, etc.

Polymers used in sustained release coatings are necessarily biocompatible, and ideally biodegradable. Examples of both naturally occurring polymers such as Aquacoat® (FMC Corporation, Food & Pharmaceutical Products Division, Philadelphia, USA) (ethylcellulose mechanically spheronised to sub-micron sized, aqueous based, pseudo-latex dispersions), and also synthetic polymers such as the Eudragit® (Röhm Pharma, Weiterstadt.) range of poly(acrylate, methacrylate) copolymers are known in the art.

### Reservoir Devices

A typical approach to modified release is to encapsulate or contain the drug entirely (e.g., as a core), within a polymer film or coat (i.e., microcapsules or spray/pan coated cores).

The various factors that can affect the diffusion process may readily be applied to reservoir devices (e.g., the effects of additives, polymer functionality (and, hence, sink-solution pH) porosity, film casting conditions, etc.) and, hence, the choice of polymer must be an important consideration in the development of reservoir devices. Modeling the release characteristics of reservoir devices (and monolithic devices) in which the transport of cephalosporin, for example cefdinir, or a salt, derivative, prodrug, or polymorph thereof, is by a solution-diffusion mechanism therefore typically involves a solution to Fick's second law (unsteady-state conditions; concentration dependent flux) for the relevant boundary conditions. When the device contains dissolved active agent, the rate of release decreases exponentially with time as the concentration (activity) of the agent (i.e., the driving force for release) within the device decreases (*i*.*e*., first order release). If, however, the active agent is in a saturated suspension, then the driving force for release is kept constant until the device is no longer saturated. Alternatively the release-rate kinetics may be desorption controlled, and a function of the square root of time.

Transport properties of coated tablets, may be enhanced compared to free-polymer films, due to the enclosed nature of the tablet core (permeant) which may enable the internal build-up of an osmotic pressure which will then act to force the permeant out of the tablet.

The effect of de-ionized water on salt containing tablets coated in poly(ethylene glycol) (PEG)-containing silicone elastomer, and also the effects of water on free films has been investigated. The release of salt from the tablets was found to be a mixture of diffusion through water filled pores, formed by hydration of the coating, and osmotic pumping. KCl transport through films containing just 10% PEG was negligible, despite extensive swelling observed in similar free films, indicating that porosity was necessary for the release of the KCl which then occurred by trans-pore diffusion. Coated salt tablets, shaped as disks, were found to swell in de-ionized water and change shape to an oblate spheroid as a result of the build-up of internal hydrostatic pressure: the change in shape providing a means to measure the force generated. As might be expected, the osmotic force decreased with increasing levels of PEG content. The lower PEG levels allowed water to be imbibed through the hydrated polymer, while the porosity resulting from the coating dissolving at higher levels of PEG content (20 to 40%) allow the pressure to be relieved by the flow of KCl.

Methods and equations have been developed, which by monitoring (independently) the release of two different salts (e.g., KCl and NaCl) allowed the calculation of the relative magnitudes that both osmotic pumping and trans-pore diffusion contributed to the release of salt from the tablet. At low PEG levels, osmotic flow was increased to a greater extent than was trans-pore diffusion due to the generation of only a low pore number density: at a loading of 20%, both mechanisms contributed approximately equally to the release. The build-up of hydrostatic pressure, however, decreased the osmotic inflow, and osmotic pumping. At higher loadings of PEG, the hydrated film was more porous and less resistant to outflow of salt. Hence, although the osmotic pumping increased (compared to the lower loading), trans-pore diffusion was the dominant release mechanism. An osmotic release mechanism has also been reported for microcapsules containing a water soluble core.

### Monolithic Devices (Matrix Devices)

Monolithic (matrix) devices may be used for controlling the release of a drug. This is possibly because they are relatively easy to fabricate compared to reservoir devices, and the danger of an accidental high dosage that could result from the rupture of the membrane of a reservoir device is not present. In such a device, the active agent is present as a dispersion within the polymer matrix, and they are typically formed by the compression of a polymer/drug mixture or by dissolution or melting. The dosage release properties of monolithic devices may be dependent upon the solubility of the drug in the polymer matrix or, in the case of porous matrixes, the solubility in the sink solution within the particle's pore network, and also the tortuosity of the network (to a greater extent than the permeability of the film), dependent on whether the drug is dispersed in the polymer or dissolved in the polymer. For low loadings of drug (0 to 5% W/V), the drug will be released by a solution-diffusion mechanism (in the absence of pores). At higher loadings (5 to 10% W/V), the release mechanism will be complicated by the presence of cavities formed near the surface of the device as the drug is lost: such cavities fill with fluid from the environment increasing the rate of release of the drug.

It is common to add a plasticizer (e.g., a poly(ethylene glycol)), a surfactant, or adjuvant (i.e., an ingredient which increases effectiveness), to matrix devices (and reservoir devices) as a means to enhance the permeability (although, in contrast, plasticizers may be fugitive, and simply serve to aid film formation and, hence, decrease permeability - a property normally more desirable in polymer paint coatings). It was noted that the leaching of PEG increased the permeability of (ethyl cellulose) films linearly as a function of PEG loading by increasing the porosity, however, the films retained their barrier properties, not permitting the transport of electrolyte. It was deduced that the enhancement of their permeability was as a result of the effective decrease in thickness caused by the PEG leaching. This was evidenced from plots of the cumulative permeant flux per unit area as a function of time and film reciprocal thickness at a PEG loading of 50% W/W: plots showing a linear relationship between the rate of permeation and reciprocal film thickness, as expected for a (Fickian) solution-diffusion type transport mechanism in a homogeneous membrane. Extrapolation of the linear regions of the graphs to the time axis gave positive intercepts on the time axis: the magnitude of which decreased towards zero with decreasing film thickness. These changing lag times were attributed to the occurrence of two diffusional flows during the early stages of the experiment (the flow of the drug and also the flow of the PEG), and also to the more usual lag time during which the concentration of permeant in the film is building-up. Caffeine, when used as a permeant, showed negative lag times. No explanation of this was forthcoming, but it was noted that caffeine exhibited a low partition coefficient in the system, and that this was also a feature of aniline permeation through polyethylene films which showed a similar negative time lag.

The effects of added surfactants on (hydrophobic) matrix devices has been investigated. It was thought that surfactant may increase the release rate of a drug by three possible mechanisms: (i) increased solubilization, (ii) improved 'wettability' to the dissolution media, and (iii) pore formation as a result of surfactant leaching. For the system studied (Eudragit® RL 100 and RS 100 plasticized by sorbitol, flurbiprofen as the drug, and a range of surfactants) it was concluded that improved wetting of the tablet led to only a partial improvement in drug release (implying that the release was diffusion, rather than dissolution, controlled), although the effect was greater for Eudragit® RS than Eudragit® RL, while the greatest influence on release was by those surfactants that were more soluble due to the formation of disruptions in the matrix allowing the dissolution medium access to within the matrix. This is of obvious relevance to a study of latex films which might be suitable for pharmaceutical coatings, due to the ease with which a polymer latex may be prepared with surfactant as opposed to surfactant-free. Differences were found between the two polymers with only the Eudragit® RS showing interactions between the anionic/cationic surfactant and drug. This was ascribed to the differing levels of quaternary ammonium ions on the polymer.

Composite devices consisting of a polymer/drug matrix coated in a polymer containing no drug also exist. Such a device was constructed from aqueous Eudragit® lattices, and was found to provide a continuous release by diffusion of the drug from the core through the shell. Similarly, a polymer core containing the drug has been produced and coated with a shell that was eroded by gastric fluid. The rate of release of the drug was found to be relatively linear (a function of the rate limiting diffusion process through the shell) and inversely proportional to the shell thickness, whereas the release from the core alone was found to decrease with time.

### Microspheres

Methods for the preparation of hollow microspheres have been described. Hollow microspheres were formed by preparing a solution of ethanol/dichloromethane containing the drug and polymer. On pouring into water, an emulsion is formed containing the dispersed polymer/drug/solvent particles, by a coacervation-type process from which the ethanol rapidly diffused precipitating polymer at the surface of the droplet to give a hard-shelled particle enclosing the drug dissolved in the dichloromethane. A gas phase of dichloromethane was then generated within the particle which, after diffusing through the shell, was observed to bubble to the surface of the aqueous phase. The hollow sphere, at reduced pressure, then filled with water which could be removed by a period of drying. No drug was found in the water. Highly porous matrix-type microspheres have also been described. The matrix-type microspheres were prepared by dissolving the drug and polymer in ethanol. On addition to water, the ethanol diffused from the emulsion droplets to leave a highly porous particle. A suggested use of the microspheres was as floating drug delivery devices for use in the stomach.

### Pendent devices

A means of attaching a range of drugs such as analgesics and antidepressants, etc., by means of an ester linkage to poly(acrylate) ester latex particles prepared by aqueous emulsion polymerization has been developed. These lattices, when passed through an ion exchange resin such that the polymer end groups were converted to their strong acid form, could self-catalyze the release of the drug by hydrolysis of the ester link.

Drugs have been attached to polymers, and also monomers have been synthesized with a pendent drug attached. Dosage forms have been prepared in which the drug is bound to a biocompatible polymer by a labile chemical bond e.g., polyanhydrides prepared from a substituted anhydride (itself prepared by reacting an acid chloride with the drug: methacryloyl chloride and the sodium salt of methoxy benzoic acid) were used to form a matrix with a second polymer (Eudragit® RL) which released the drug on hydrolysis in gastric fluid. The use of polymeric Schiff bases suitable for use as carriers of pharmaceutical amines has also been described.

### Enteric films

Enteric coatings consist of pH sensitive polymers. Typically the polymers are carboxylated and interact very little with water at low pH, while at high pH the polymers ionize causing swelling or dissolving of the polymer. Coatings can therefore be designed to remain intact in the acidic environment of the stomach, protecting either the drug from this environment or the stomach from the drug, but to dissolve in the more alkaline environment of the intestine.

### Osmotically controlled devices

The osmotic pump is similar to a reservoir device but contains an osmotic agent (e.g., the active agent in salt form) which acts to imbibe water from the surrounding medium via a semi-permeable membrane. Such a device, called an elementary osmotic pump, has been described. Pressure is generated within the device which forces the active agent out of the device via an orifice of a size designed to minimize solute diffusion, while preventing the build-up of a hydrostatic pressure head which can have the effect of decreasing the osmotic pressure and changing the dimensions of the device. While the internal volume of the device remains constant, and there is an excess of solid or saturated solution in the device, then the release rate remains constant delivering a volume equal to the volume of solvent uptake.

### Electrically stimulated release devices

Monolithic devices have been prepared using polyelectrolyte gels which swell when, for example, an external electrical stimulus is applied causing a change in pH. The release may be modulated by changes in the applied current to produce a constant or pulsatile release profile.

### Hydrogels

In addition to their use in drug matrices, hydrogels find use in a number of biomedical applications such as, for example, soft contact lenses, and various soft implants, and the like.

### Methods of Using Modified Release Compositions Comprising Cephalosporin

According to another aspect of the present invention, there is provided a method for treating a patient suffering from a bacterial disorder comprising the step of administering a therapeutically effective amount of the composition of the present invention in solid oral dosage form. Advantages of the method of the present invention include a reduction in the dosing frequency required by conventional multiple IR dosage regimes while still maintaining the benefits derived from a pulsatile plasma profile or eliminating or minimizing the variations in plasma concentration levels. This reduced dosing frequency is advantageous in terms of patient compliance and the reduction in dosage frequency made possible by the method of the present invention would contribute to controlling health care costs by reducing the amount of time spent by health care workers on the administration of cephalosporin, for example cefdinir, or a salt, derivative, prodrug, or polymorph thereof.

In the following examples, all percentages are weight by weight unless otherwise stated. The term "purified water" as used throughout the Examples refers to water that has been purified by passing it through a water filtration system. It is to be understood that the examples are for illustrative purposes only, and should not be interpreted as restricting the spirit and breadth of the invention as defined by the scope of the claims that follow.

### Examples

Examples 1 to 4 provide exemplary cefdinir tablet formulations. These examples are not intended to limit the claims in any respect, but rather to provide exemplary tablet formulations of cefdinir which can be utilized in the methods of the invention. Such exemplary tablets can also comprise or carry a coating agent. These ranges for the individual ingredients are also proposed herein as individual preferred ranges for those components considered separately of the others listed, and also for other components of the above-mentioned category to which they belong.

### Example 1

| **Exemplary Nanoparticulate Cefdinir Tablet Formulation #1** | |
|---|---|
| **Component** | **g/Kg** |
| Cefdinir | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

### Example 2

| **Exemplary Nanoparticulate Cefdinir Tablet Formulation #2** | |
|---|---|
| **Component** | **g/Kg** |
| Cefdinir | about 100 to about 300 |
| Hypromellose, USP | about 30 to about 50 |
| Docusate Sodium, USP | about 0.5 to about 10 |
| Sucrose; NF | about 100 to about 300 |
| Sodium Lauryl Sulfate, NF | about 1 to about 30 |
| Lactose Monohydrate, NF | about 100 to about 300 |
| Silicified Microcrystalline Cellulose | about 50 to about 200 |
| Crospovidone, NF | about 50 to about 200 |
| Magnesium Stearate, NF | about 0.5 to about 5 |

### Example 3

| **Exemplary Nanoparticulate Cefdinir Tablet Formulation #3** | |
|---|---|
| **Component** | **g/Kg** |
| Cefdinir | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

### Example 4

| **Exemplary Nanoparticulate Cefdinir Tablet Formulation #4** | |
|---|---|
| **Component** | **g/Kg** |
| Cefdinir | about 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose | about 129 to about 134 |
| Crospovidone, NF | about 112 to about 118 |
| Magnesium Stearate, NF | about 0.5 to about 3 |

### Example 5

### Multiparticulate Modified Release Composition Containing Cefdinir

A multiparticulate modified release composition according to the present invention comprising an immediate release component and a modified release component containing cephalosporin is prepared as follows.

### (a) Immediate Release Component.

A solution of cefdinir (50:50 racemic mixture) is prepared according to any of the formulations given in Table 1. The methylphenidate solution is then coated onto nonpareil seeds to a level of approximately 16.9% solids weight gain using, for example, a Glatt GPCG3 (Glatt, Protech Ltd., Leicester, UK) fluid bed coating apparatus to form the IR particles of the immediate release component.

**TABLE 1**

| Immediate release component solutions | | |
|---|---|---|
| | Amount, | % (w/w) |
| Ingredient | (i) | (ii) |
| Cefdinir | 13.0 | 13.0 |
| Polyethylene Glycol 6000 | 0.5 | 0.5 |
| Polyvinylpyrrolidone | 3.5 | |
| Purified Water | 83.5 | 86.5 |

### (b) Modified Release Component

Cefdinir -containing delayed release particles are prepared by coating immediate release particles prepared according to Example 1 (a) above with a modified release coating solution as detailed in Table 2. The immediate release particles are coated to varying levels up to approximately to 30% weight gain using, for example, a fluid bed apparatus.

**TABLE 2**

| Modified release component coating solutions | | | | | | | | |
|---|---|---|---|---|---|---|---|---|
| Amount, % (w/w) | | | | | | | | |
| Ingredient | (i) | (ii) | (iii) | (iv) | (v) | (vi) | (vii) | (viii) |
| Eudragit ® RS 12.5 | 49.7 | 42.0 | 47.1 | 53.2 | 40.6 | -- | -- | 25.0 |
| Eudragit ® S 12.5 | -- | -- | -- | -- | -- | 54.35 | 46.5 | -- |
| Eudragit ® L 12.5 | -- | -- | -- | -- | -- | -- | 25.0 | |
| Polyvinylpyrrolidone | -- | -- | -- | 0.35 | 0.3 | -- | -- | |
| Diethylphthalate | 0.5 | 0.5 | 0.6 | 1.35 | 0.6 | 1.3 | 1.1 | -- |
| Triethylcitrate | -- | -- | -- | -- | -- | -- | -- | 1.25 |
| Isopropyl alcohol | 39.8 | 33.1 | 37.2 | 45.1 | 33.8 | 44.35 | 49.6 | 46.5 |
| Acetone | 10.0 | 8.3 3 | 9.3 .3 | -- | 8.4 .4 | -- | -- | -- |
| Talc¹ | -- | 16.0 | 5.9 | -- | 16.3 | -- | 2.8 | 2.25 |

| | | | | | | | | |
|---|---|---|---|---|---|---|---|---|
| ¹Talc is simultaneously applied during coating for formulations in column (i), (iv) and (vi). | | | | | | | | |

### (c) Encapsulation of Immediate and Delayed Release Particles.

The immediate and delayed release particles prepared according to Example 5(a) and (b) above are encapsulated in size 2 hard gelatin capsules to an overall 20 mg dosage strength using, for example, a Bosch GKF 4000S encapsulation apparatus. The overall dosage strength of 20 mg cephalosporin was made up of 10 mg from the immediate release component and 10 mg from the modified release component.

### Example 6

### Multiparticulate Modified Release Composition Containing Cefdinir

Multiparticulate modified release cephalosporin compositions according to the present invention having an immediate release component and a modified release component having a modified release matrix material are prepared according to the formulations shown in Table 5(a) and (b).

**TABLE 5 (a)**

| 100 mg of IR component is encapsulated with 100 mg of modified release (MR) component to give a 20 mg dosage strength product | |
|---|---|
| IR component | % (w/w) |
| Cefdinir | 10 |
| Microcrystalline cellulose | 40 |
| Lactose | 45 |
| Povidone | 5 |

| MR component | |
|---|---|
| Cefdinir | 10 |
| Microcrystalline cellulose | 40 |
| Eudragit ® RS | 45 |
| Povidone | 5 |

**TABLE 5 (b)**

| 50 mg of IR component is encapsulated with 50 mg of modified release (MR) component to give a 20 mg dosage strength product. | |
|---|---|
| | % (w/w) |
| IR component | |
| Cefdinir | 20 |
| Microcrystalline cellulose | 50 |
| Lactose | 28 |
| Povidone | 2 |

| MR component | |
|---|---|
| Cefdinir | 20 |
| Microcrytalline cellulose | 50 |
| Eudragit ® S | 28 |
| Povidone | 2 |

### Example 7

The purpose of this prophetic example was describe how a nanoparticulate cephalosporin composition could be prepared.

An aqueous dispersion of 5% (w/w) cefdinir, combined with one or more surface stabilizers, such as hydroxypropyl cellulose (HPC-SL) and dioctylsulfosuccinate (DOSS), could be milled in a 10 ml chamber of a NanoMill® 0.01 (NanoMill Systems, King of Prussia, PA; see e.g., U.S. Patent No. 6,431,478), along with 500 micron PolyMill® attrition media (Dow Chemical Co.) (e.g., at an 89% media load). In an exemplary process, the mixture could be milled at a speed of 2500 rpms for 60 minutes.
Following milling, the particle size of the milled cefdinir particles can be measured, in deionized distilled water, using a Horiba LA 910 particle size analyzer. For a successful composition, the initial mean and/or D50 milled cefdinir particle size is expected to be less than 2000 nm.

It will be apparent to those skilled in the art that various modifications and variations can be made in the methods and compositions of the present inventions without departing from the spirit or scope of the invention. Thus, it is intended that the present invention cover the modification and variations of the invention provided they come within the scope of the appended claims and their equivalents.

## Claims

1. A stable nanoparticulate composition comprising: (A) particles comprising a cephalosporin, said particles having an effective average particle size of less than about 2000 nm in diameter; and (B) at least one surface stabilizer.

2. The composition of claim 1, wherein said cephalosporin is cefdinir or a salt, derivative, prodrug, or polymorph thereof, said cefdinir being present either as a single substantially optically pure enantiomer thereof or as a mixture of enantiomers thereof.

3. Composition of claim 1 or 2, wherein said particles are in a crystalline phase, an amorphous phase, a semi-crystalline phase, a semi amorphous phase, or a mixture thereof.

4. Composition of claim 1, 2 or 3 further comprising one or more pharmaceutically acceptable excipients, carriers, or a combination thereof.

5. Composition of any one of the preceding claims wherein the surface stabilizer is selected from a non-ionic surface stabilizer, an anionic surface stabilizer, a cationic surface stabilizer, a zwitterionic surface stabilizer, and an ionic surface stabilizer.

6. Composition of claim 2 or claim dependent thereon, comprising:
(a) about 50 to about 500 g/kg cefdinir;
(b) about 10 to about 70 g/kg hypromellose;
(c) about 1 to about 10 g/kg docusate sodium;
(d) about 100 to about 500 g/kg sucrose;
(e) about 1 to about 40 g/kg sodium lauryl sulfate;
(f) about 50 to about 400 g/kg lactose monohydrate;
(g) about 50 to about 300 g/kg silicified microcrystalline cellulose;
(h) about 20 to about 300 g/kg crospovidone; and
(i) about 0.5 to about 5 g/kg magnesium stearate.

7. Composition of any one of claims 2 to 5 comprising:
(a) about 100 to about 300 g/kg cefdinir;
(b) about 30 to about 50 g/kg hypromellose;
(c) about 0.5 to about 10 g/kg docusate sodium;
(d) about 100 to about 300 g/kg sucrose;
(e) about 1 to about 30 g/kg sodium lauryl sulfate;
(f) about 100 to about 300 g/kg lactose monohydrate;
(g) about 50 to about 200 g/kg silicified microcrystalline cellulose;
(h) about 50 to about 200 g/kg crospovidone; and
(i) about 0.5 to about 5 g/kg magnesium stearate.

8. The composition of claim 6 or 7, further comprising a coating agent.

9. Composition of any one of the preceding claims comprising one or more additional active compounds useful for the prevention and treatment of a bacterial infection.

10. Composition of claim 2 or claim dependent thereon wherein said particles contain a reservoir which contains cefdinir, or a salt, derivative, prodrug, or polymorph thereof, said reservoir being enclosed by a semi-permeable membrane which allows for water to be imbibed into said particles, thus generating pressure which forces said cephalosporin out of said particles.

11. The composition of claim 10 wherein said reservoir comprises also an osmotic agent.

12. A method of preparing a composition of any one of claims 1 to 11 comprising contacting particles comprising cephalosporin (e.g. cefdinir, or a salt, derivative, prodrug, or polymorph thereof), with at least one surface stabilizer for a period of time and under conditions sufficient to provide a nanoparticulate composition comprising the cephalosporin (e.g. cefdinir, or a salt, derivative, prodrug, or polymorph thereof) having an effective average particle size of less than about 2000 nm in diameter.

13. A pharmaceutical composition comprising a first component of active ingredient-containing particles and at least one subsequent component of active ingredient-containing particles, wherein at least one of said components comprises particles wherein a cephalosporin is the active ingredient and at least one of said components further comprises a modified release coating, a modified release matrix material, or both, such that the composition, following oral delivery to a subject, delivers the active ingredient in a continuous, bimodal or multimodal manner.

14. A composition of claim 13 wherein said cephalosporin is cefdinir or a salt, derivative, prodrug, or polymorph thereof.

15. The composition of claim 14 wherein the particles containing cefdinir or a salt, derivative, prodrug, or polymorph thereof comprise nanoparticles which comprise said cefdinir or a salt, derivative, prodrug, or polymorph thereof.

16. Composition of claim 14 or 15 wherein the particles containing cefdinir or a salt, derivative, prodrug, or polymorph thereof are nanoparticles which comprise said cefdinir or a salt, derivative, prodrug, or polymorph thereof.

17. Composition of claim 14, 15 or 16 wherein each component comprises particles in which the active ingredient is cefdinir or a salt, derivative, prodrug, or polymorph thereof.

18. Composition of any one of claims 13 to 17 wherein the first component comprises an immediate release component and at least one subsequent component comprises a modified release component.

19. Composition of any one of claims 13 to 18 wherein the active ingredient-containing particles are erodable.

20. Composition of any one of claims 13 to 19 wherein said composition further comprises an enhancer.

21. A dosage form comprising a composition of any one of the preceding claims.

22. The dosage form of claim 21 comprising a blend of active ingredient-containing particles contained within a hard gelatin or soft gelatin capsule.

23. The dosage form of claim 22, wherein the active ingredient-containing particles are in the form of mini-tablets and the capsule contains a mixture of said mini-tablets.

24. The dosage form of claim 21 or 22 wherein the particles comprising cefdinir, or a salt, derivative, prodrug, or polymorph thereof, are provided in a rapidly dissolving dosage form.

25. The dosage form of claim 21 in the form of tablet.

26. The dosage form of claim 25 wherein the tablet is a fast-melt tablet.

27. The composition of any one of claims 13 to 20 wherein the modified-release coating comprises a pH-dependent polymer coating for releasing a pulse of the active ingredient in said subject following a time delay of about 6 to about 12 hours after administration of said composition to said subject.
